# EUROPEAN PATENT SPECIFICATION

(11) **EP 0 994 119 B1**
(45) Date of publication and mention of the grant of the patent: **31.03.2004**
(21) Application number: 99120330.8
(22) Date of filing: 12.10.1999
(51) Int. Cl.: C07J 71/00, A61K 31/59

(54) **Stereoselective process for the preparation of the 22R epimer of budesonide**
Stereoselektives Verfahren zur Herstellung vom 22R Epimer von Budesonid
Procédé stereoselectif pour la préparation de l'épimer 22R de budesonide

(30) Priority: 13.10.1998 IT MI982196
(43) Date of publication of application: 19.04.2000
(73) Proprietor: Farmabios S.p.A., 27027 Gropello Cairoli (Pavia) (IT)
(72) Inventor: La Loggia, Filippo, 27027 Gropello Cairoli (PAVIA) (IT); Dotti, PierFranco, 20010 Vittuone (MILAN) (IT)
(74) Representative: Gervasi, Gemma, Dr.

(56) References cited:
- EP-A- 0 164 636
- EP-A- 0 262 108
- EP-A- 0 508 900
- EP-A- 0 875 516

## Description

### Field of the invention

The present invention relates to a stereoselective process for the preparation of budesonide, a glucocorticoid with anti-inflammatory activity, in the form of the 22R epimer, which, is pharmacologically more active than the corresponding 22S epimer.

### Prior art

Due to the presence of acetal carbon at the C-22 position, budesonide, i.e. [16,17-butylidenebis(oxy)-11,21-dihydroxy-pregna-1,4-diene-3,20-dione], exists in the form of epimer at the C-22 position. The 22R epimer of budesonide, sometimes also referred to as epimer B, is the most active from a pharmacological point of view.

This is the reason why continued efforts have been made to develop a stereoselective process for the preparation of the 22R epimer as well as separation processes for isolating high-purity 22R budesonide from mixtures obtained by non-stereoselective processes, which contain both the 22R and the 22S epimers.

EP-569 369 describes a method for the preparation of 22R budesonide starting from 11β,16α,17α,21-tetrahydroxy-1,4-pregnadiene-3,2-dione 21-acetate by treatment with butyraldehyde in 70-80% aqueous HF, followed by hydrolysis of the acetate to budesonide.

EP-A-164 636 describes the preparation of budesonide and of structurally correlated 16,17-pregnane acetals in the form of mixtures in which the R epimer is preponderant, by transketalisation of the corresponding 16-α,17α-acetonides (or 16,17-diols) of 16-α-hydroxyprednisolone with butyraldehyde in aqueous HF or HCl in concentrations not higher than 90%.

The use of HF, highly corrosive, requires special equipment, which involves higher production costs; moreover, it arouses serious environmental problems related to its disposal, especially when used in very high concentrations, as in the processes described in EP-569 369 and EP-A-164 636.

As may be inferred from the Examples of EP-A-164 636, mixtures of the two epimers are always obtained, in which the R epimer never exceeds 90%. When operating in the presence of aqueous HCl, yields do not improve and the product obtained is less pure.

EP-A-262 108 describes a method for the preparation of epimeric mixtures of 11β-hydroxy-16,17-acetals of pregnane derivatives by reaction of the corresponding 16,17-diols or 16,17-acetonides with carbonyl compounds in the presence of acid catalysts, preferably perchloric acid, in hydrocarbon solvent, e.g. isooctane, or halogenated hydrocarbon solvent, such as chloroform or methylene chloride.

EP-A-262 108 also describes a method for preparing predominantly of the 22R epimer: the reaction is carried out in the presence of high quantities (approx. 20 times by wt. in respect of the organic substrate) of granular material (e.g. sand or ceramic material) in fine particles. In any case, the reaction is carried out in the presence of one of the aforementioned organic solvents, whose use, due to their toxicity, is to be avoided.

GB 1,429,922 describes the synthesis of budesonide and its structural analogues by treatment of the corresponding 16α,17α-diols of 11β,21-dihydroxy-pregna-1,4-diene-3,20-diones with aldehydes in dioxane in the presence of perchloric acid.

This method yields mixtures of epimers at the C-22 position: their separation is difficult to perform and may be obtained only by techniques, such as gel filtration, as described in USP 3,928,326 or in GB 1,428,416, which can be hardly scaled-up.

EP-A-508,900 discloses the transketalisation and dehalogenation of 92-Halo desonide 11-β-formyloxy compounds.

It is, therefore, felt the need for a stereoselective process for the preparation of the R22 epimer of budesonide, not requiring time-consuming and expensive purification procedures and being free from the drawbacks of the processes known in the art.

### Summary of the invention

The Applicant surprisingly succeeded in obtaining the 22R epimer of budesonide having a purity higher than 90% by transketalisation of a particular substrate, the desonide derivative of formula (II) reported hereinafter, with butyraldehyde in the presence of an aqueous halohydric acid selected from HBr and HI. If necessary, the reaction product may be further enriched in the desired 22R epimer by one or more recrystallisation procedures.

The Applicant has moreover unexpectedly found that the ratio between epimers at the C-22 position keeps substantially unaltered when the aforesaid reaction product is subjected to dehalogenation to give the final budesonide of formula (I) shown below.

It is, therefore, object of the present invention a stereoselective process for the preparation of budesonide in the form of 22R epimer having purity higher than 90%, said process comprising the following steps:
a) stereoselective transketalisation of the desonide derivative of formula 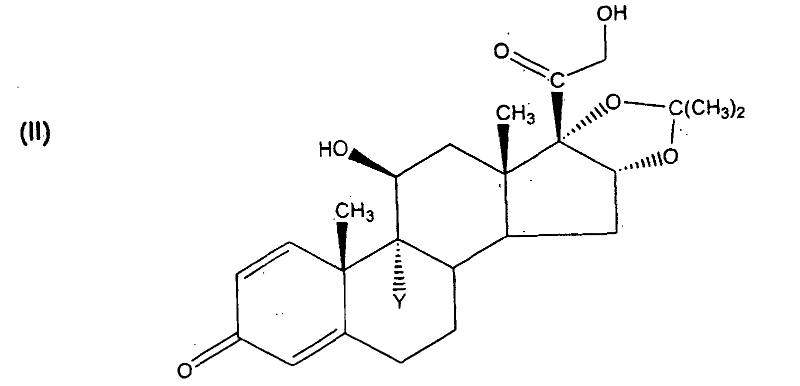 wherein Y is selected between Br and I, by reaction with butyraldehyde in an aqueous halohydric acid selected between HBr and Hl, to give the compound of formula (III) 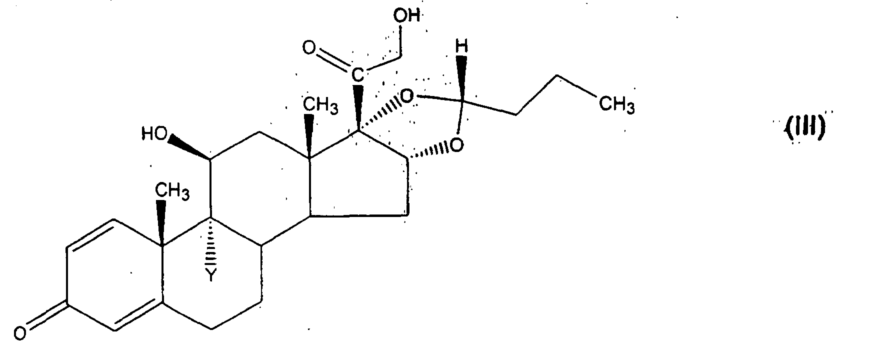 wherein Y has the meaning above reported;
b) treatment of 9α-bromo-budesonide or 9α-iodo-budesonide of formula (III) from step a) with a dehalogenating agent, to give budesonide of formula (I).

Features and advantages of the process according to the present invention will be illustrated in detail in the following description.

### Detailed description of the invention

The present process allows the obtainment of budesonide having an R/S ratio of epimers at the C-22 position higher than 90:10.

In particular, the Applicant has found that, in the transketalisation conditions in acqueous HBr or Hl according to the present invention, budesonide with an R/S ratio higher than 90:10 generally forms in a period shorter than 10 hours.

Compared with the transketalisation in aqueous HF of the prior art, the reaction in aqueous HBr and HI of the present invention is surprisingly advantageous, as it gives high-purity 22R budesonide in high yields and it secures greater reliability and safety of the industrial-scale process, thanks to the lower environmental impact and to the higher solvent power of aqueous HBr. Instead, the prior art suggested a decreased power of halohydric acids from HF to higher molecular weight acids.

According to typical embodiments of the present invention, step a) utilises, as a transketalisation substrate, halohydrin of formula (II), in which Y is selected from between Br and I, which may be easily prepared from commercial products, to obtain the corresponding compound of formula (III), 9α-bromo-budesonide or 9α-iodo-budesonide, which is subjected to dehalogenation in step b) to give budesonide of formula (I).

According to a preferred embodiment of the present invention, the transketalisation substrate consists of the compound of formula (II) in which Y is Br, i.e. 9α-bromo-desonide.

Step a) is carried out by causing the substrate of formula (II) to react with butyraldehyde in a molar ratio preferably ranging between 1:1 and 1:5, and more preferably between 1:3 and 1:4, expressed as moles of aldehyde with respect to moles of substrate of formula (II).

Said step a) is carried out in the presence of an aqueous halohydric acid selected from between HBr and Hl, in a concentration ranging from 20% to 70% by weight. Aqueous HBr in a concentration of 48% to 62% approx., typically of 48% approx., or aqueous Hl in a concentration of 47% to 67% approx., typically of 55% to 57% approx., corresponding to the products available on the market, are preferably used.

The transketalisation reaction of step a) is carried out preferably at a temperature ranging from 10°C to 20°C, more preferably at 20°C.

Said reaction is generally carried out using 1 to 20 parts by vol. of aqueous HBr or Hl per part by wt. of substrate of formula (II).

According to a typical embodiment of the present invention, transketalisation is carried out exclusively in aqueous halohydric acid as a reaction solvent; in any case, also other convenient water miscible organic solvents may be optionally added.

Step a) gives 9α-bromo-budesonide or 9α-iodo-budesonide of formula (III) with a R/S ratio between epimers at the C-22 position of approx. 90:10, with reaction times shorter than 10 hours, preferably of *ca*. 5 hours.

The product of formula (III) is typically isolated from the reaction mixture by precipitation in water, pre-cooled to a temperature ranging from 0°C to 5°C, in a quantity equal to *ca*. 10 parts by vol. in respect of the volume of the reaction mixture.

With a view to enriching the product from step a) in the desired 22R epimer, it may be purified by one or more crystallisations from a convenient solvent or solvent mixtures.

The solvent preferably used in the crystallisation of compound of formula (III) is selected from the group consisting of water, methyl alcohol, methylene chloride, tetrahydrofuran, diisopropyl ether, and mixtures thereof.

The temperature at which the product to be purified is re-dissolved preferably ranges from 20°C to 40°C.

According to a preferred embodiment of the present invention, the product from step a) is purified by crystallisation from methyl alcohol/water, at a temperature of 20°C to 40°C.

The product from step a), optionally purified by crystallisation, is then subjected to dehalogenation in step b) to give the corresponding product of formula (I).

The ratio between budesonide epimers at the C-22 position achieved in the transketalisation of step a) in aqueous HBr or Hl is unexpectedly at least maintained in dehalogenation of step b), or better increased by approx. 1% in favour of the 22R epimer.

The dehalogenation reaction in step b) is typically carried out at a temperature of 50°C to 70°C.

The dehalogenating agent is typically an organotin hydride, such as tributyl tin hydride, typically used in the presence of a radical initiator, e.g. α,α '-azoisobutyronitrile or benzoyl peroxide, generally in an ethereal solvent, e.g. isopropyl ether, ethyl ether, tetrahydrofuran, or in ethyl acetate, acetonitrile, or in an aromatic solvent, e.g. toluene, or in an aprotic polar solvent, e.g. dimethylformamide.

According to a preferred embodiment of the present invention, in step b) tributyl tin hydride is used in the presence of α,α'-azoisobutyronitrile, the solvent is tetrahydrofuran and the temperature ranges from 50°C to 70°C.

The stereochemistry of the intermediate of formula (III) and of the final product of formula (I) as well as of the products obtained from purification treatments, if any, was evaluated by HPLC analysis on a convenient column.

The following examples are conveyed by way of indication, not of limitation, of the present invention.

### Example 1

### Preparation of 9α-bromo-budesonide of formula (III) starting from 9α-bromo-desonide of formula (II)

a) Transketalisation: 9α-bromo-desonide (27 g) was added under stirring at a temperature of 10°C to a mixture consisting of 48% aqueous hydrobromic acid (270 ml) and butyraldehyde (21.6 ml). The reaction was continued for 6 hrs. The reaction mass was poured into water (2,700 ml), pre-cooled to 5°C, to yield a suspension from which a solid was separated by filtration.

Purification: The solid was crushed in diisopropyl ether (270 ml) at 40°C. The crushed product was dissolved in methanol (400 ml) at 40°C and reprecipitated by addition of water (400 ml). This operation was performed twice. The solid was then dissolved in a mixture of ethyl acetate (50 ml) and tetrahydrofuran (150 ml), previously decolourised on carbon (2 g). The resulting solution was concentrated under vacuum to a volume of 40 ml. The solid was allowed to precipitate by addition of diisopropyl ether (200 ml); then, it was filtered and dried under vacuum at 40°C.

16.5 g of 9α-bromo-budesonide (yield = 59%) with an R/S ratio of 95:5 was obtained.

### Example 2

### Preparation of 9α-bromo-budesonide (III) starting from 9α-bromo-desonide (II)

a) Transketalisation: 9α-bromo-desonide (30 g) was added under stirring at a temperature of 20°C to a mixture of butyraldehyde (24 ml) with 48% aqueous hydrobromic acid (300 ml). The reaction was continued for 4 hrs. The reaction mass was poured into water (3,000 ml), pre-cooled to 5°C, to yield a suspension from which a solid was separated by filtration.

Purification: The solid was dissolved in tetrahydrofuran (150 ml) at 40°C, concentrated under vacuum to obtain an oil, whereto diisopropyl ether (150 ml) was added. By filtration a product was obtained which was then redissolved at 40°C in methanol (550 ml) and reprecipitated by addition of water (550 ml). Filtration and drying under vacuum at 40°C gave 19 g of 9α-bromo-budesonide (yield = 62%) with an R/S ratio of 97:3.

### Example 3

### Preparation of 9α-bromo-budesonide (III) starting from 9α-bromo-desonide (II)

a) Transketalisation: 9α-bromo-desonide (53 g) was added under stirring at a temperature of 20°C to 48% aqueous hydrobromic acid (530 ml). Then butyraldehyde (42.4 ml) was added dropwise. The mixture was caused to react for 2.5 hrs. The reaction mass was poured into water (4,000 ml), pre-cooled to 5°C, to yield a suspension from which a solid was separated by filtration.

Purification: The solid was dissolved in methylene chloride (500 ml) at 40°C and reprecipitated by addition of water at room temperature, followed by concentration under vacuum to small volume and addition of diisopropyl ether (500 ml). The solid was separated by filtration, and redissolved at 40°C in a mixture of methylene chloride (600 ml) and methanol (15 ml). Once the mixture had been concentrated under vacuum to small volume, it was added with diisopropyl ether (380 ml). The so-obtained solid product was filtered and dried under vacuum at 40°C to give 32.4 g of 9α-bromo-budesonide (yield = 59%) with an epimeric R/S ratio of 97:3.

### Example 4

b) Debromination: 9α-bromo-budesonide (32,4 g) prepared as per Example 3 was dissolved in tetrahydrofuran (324 ml). Then tributyl tin hydride (17.8 ml) and α,α'-azoisobutyronitrile (0.32 g) were added and the reaction mixture was refluxed for 16 hrs. Once 9α-bromo-budesonide was completely disappeared, as revealed by HPLC, the mixture was concentrated under vacuum, diluted with diisopropyl ether (250 ml), cooled to 10°C, and filtered. The solid product was washed with diisopropyl ether and, finally, air dried.

24.0 g of budesonide (yield = 88%) with an epimeric R/S ratio of 98:2 was obtained.

## Claims

1. A stereoselective process for the preparation of budesonide in the form of the 22R epimer said process comprising the following steps:
a) stereoselective transketalisation of the desonide derivative of formula (II) 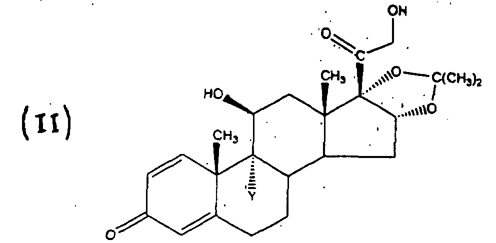 wherein Y is selected between Br and I, by reaction with butyraldehyde in the presence of a halohydric acid selected between aqueous HBr and HI, to give the compound of formula (III) wherein Y has the meaning above reported; 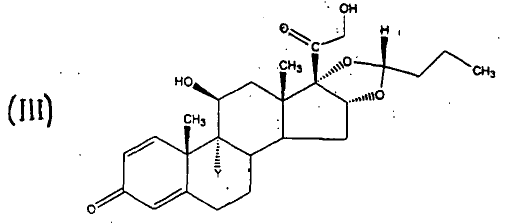
b) treatment of 9α-bromo-budesonide or 9α-iodo-budesonide of formula (III) from step a) with a dehalogenating agent, to give budesonide.

2. The process as claimed in claim 1, wherein the butyraldehyde/9α-bromo-desonide or butyraldehyde/9α-iodo-desonide molar ratio in step a) ranges from 1:1 to 1:5.

3. The process as claimed in claim 2, wherein said molar ratio ranges from 1:3 to 1:4.

4. The process as claimed in claim 1, wherein the concentration of aqueous halohydric acid in step a) ranges from 20% to 70% by weight.

5. The process as claimed in claim 4, wherein said concentration is 48% by weight when said halohydric acid is aqueous HBr, or said concentration is 55% to 57% by weight when said halohydric acid is aqueous HI.

6. The process as claimed in claim 1, wherein step a) is carried out with 1 to 20 parts by vol. of aqueous HBr or HI per part by wt. of substrate.

7. The process as claimed in claim 1, wherein step a) is carried out at a temperature of 10°C to 20°C.

8. The process as claimed in claim 1, wherein step a) is carried out with a reaction time of 2 to 10 hrs.

9. The process as claimed in claim 1, wherein the product 9α-bromo-budesonide or 9α-iodo-budesonide from step a) is purified by re-crystallisation from a solvent selected from a group consisting of water, methyl alcohol, methylene chloride, tetrahydrofuran, diisopropyl ether, and mixtures thereof.

10. The process as claimed in claim 9, wherein said solvent is a methyl alcohol/water mixture.

11. The process as claimed in claim 1, wherein dehalogenation step b) is carried out at a temperature of 50 to 70°C.

12. The process as claimed in claim 1, wherein step b) is carried out with an organotin hydride in the presence of a radical initiator.

13. The process as claimed in claim 12, wherein said organotin hydride is tributyl tin hydride and said radical initiator is α,α'-azoisobutyronitrile.

## Patentansprüche

1. Ein stereoselektives Verfahren zur Herstellung von Budesonid in Form seines 22R-Epimers, wobei dieses Verfahren die folgenden Schritte umfasst:
a) stereoselektive Transketalisierung des Desonidderivats mit der Formel II 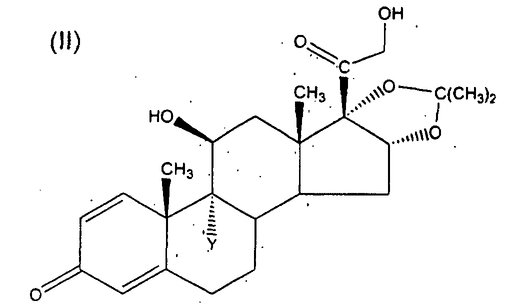 wobei Y ausgewählt wird aus Br und 1, durch Reaktion mit Butyraldehyd in Gegenwart einer Halogenwasserstoffsäure, ausgewählt aus wässrigem HBr und HI, um eine Verbindung mit der Formel III zu ergeben, wobei Y die obenstehende Bedeutung besitzt; 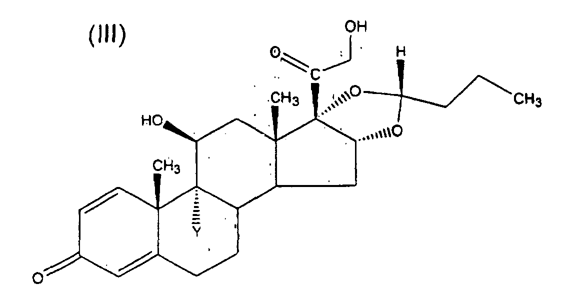
b) Behandlung von 9α-Brombudesonid oder 9α-Iodbudesonid der Formel III aus Schritt a) mit einem dehalogenierendem Reagenz, um Budesonid zu erhalten.

2. Das Verfahren gemäß Anspruch 1, wobei das Molverhältnis von Butyraldehyd/9α-Bromdesonid oder von Butyraldehyd/9α-Ioddesonid in Schritt a) zwischen 1:1 und 1:5 liegt.

3. Das Verfahren gemäß Anspruch 2, wobei das Molverhältnis zwischen 1:3 und 1: 4 liegt.

4. Das Verfahren gemäß Anspruch 1, wobei die Konzentration der wässrigen Halogenwasserstoffsäure in Schritt a) zwischen 20 Gew.-% und 70 Gew.-% beträgt.

5. Das Verfahren gemäß Anspruch 4, wobei die Konzentration 48 Gew.-% beträgt, wenn die Halogenwasserstoffsäure wässrige HBr ist, oder die Konzentration 55 Gew.-% bis 57 Gew.-% beträgt, wenn die Halogenwasserstoffsäure wässriges HI ist.

6. Das Verfahren gemäß Anspruch 1, wobei Schritt a) mit 1 bis 20 Volumenteilen wässriger HBr oder Hl pro Gewichtsteil Substrat ausgeführt wird.

7. Das Verfahren gemäß Anspruch 1, wobei Schritt a) bei einer Temperatur von 10 °C bis 20 °C ausgeführt wird.

8. Das Verfahren gemäß Anspruch 1, wobei Schritt a) mit einer Reaktionszeit, von 2 bis 10 Stunden ausgeführt wird.

9. Das Verfahren gemäß Anspruch 1, wobei die Produkte 9α-Brombudesonid oder 9α-lodbudesonid aus Schritt a) durch Umkristallisation aus einem Lösungsmittel, ausgewählt aus der Gruppe bestehend aus Wasser, Methanol, Methylenchlorid, Tetrahydrofuran, Dilsopropylether und Mischungen daraus, gereinigt wird.

10. Das Verfahren gemäß Anspruch 9, wobei das Lösungsmittel eine Methanol/Wasser-Mischung ist.

11. Das Verfahren gemäß Anspruch 1, wobei der Dehalogenierungsschritt b) bei einer Temperatur von 50 bis 70 °C ausgeführt wird.

12. Das Verfahren gemäß Anspruch 1, wobei Schritt b) mit einem Organozinnhydrid in Gegenwart eines Radikalstarters ausgeführt wird.

13. Das Verfahren gemäß Anspruch 12, wobei das Organozinnhydrid Tributylzinnhydrid und der Radikalstarter α,α'-Azoisobuttersäurenitril ist.

## Revendications

1. Procédé stéréosélectif pour la préparation de budésonide sous la forme de l'épimère 22R, ledit procédé comprenant les étapes suivantes :
(a) transcétalisation stéréosélective du dérivé désonide de formule (II) : 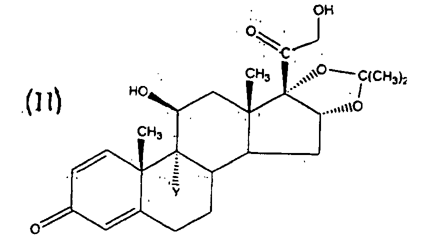 dans laquelle Y est choisi entre Br et l, par réaction avec du butyraldéhyde en présence d'un acide halohydrique choisi entre HBr et HI aqueux, pour donner le composé de formule (III) dans laquelle Y a la signification donnée plus haut : 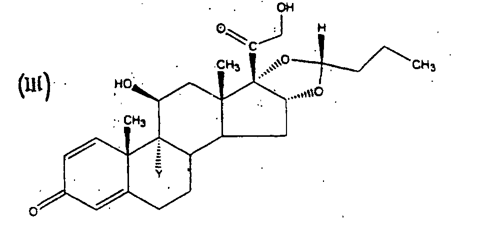
(b) traitement du 9α-bromo-budésonide ou du 9α-iodo-budésonide de formule (III) provenant de l'étape (a) avec un agent de déshalogénation pour donner un budésonide.

2. Procédé selon la revendication 1, dans lequel le rapport molaire butyraldéhyde/9α-bromo-désonide ou butyraldéhyde/9α-iodo-désonide dans l'étape (a) est compris dans la gamme de 1 :1 à 1 :5.

3. Procédé selon la revendication 2, dans lequel ledit rapport molaire est compris dans la gamme de 1 :3 à 1 :4.

4. Procédé selon la revendication 1, dans lequel la concentration de l'acide halohydrique aqueux dans l'étape (a) est comprise dans la gamme de 20% à 70% en poids.

5. Procédé selon la revendication 4, dans lequel ladite concentration est de 48% en poids lorsque ledit acide halohydrique est du HBr aqueux, ou ladite concentration est 55% à 57% en poids lorsque ledit acide halohydrique est du Hl aqueux.

6. Procédé selon la revendication 1, dans lequel l'étape (a) est effectuée avec 1 à 20 parties en volume de HBr ou de HI aqueux par partie en poids du substrat.

7. Procédé selon la revendication 1, dans lequel l'étape (a) est effectuée à une température de 10°C à 20°C.

8. Procédé selon la revendication 1, dans lequel l'étape (a) est effectuée avec une durée de réaction de 2 à 10 heures.

9. Procédé selon la revendication 1, dans lequel le 9α-mo-budésonide ou le 9α-iodo-budésonide provenant de l'étape (a) est purifié par recristallisation dans un solvant choisi dans le groupe consistant en eau, alcool méthylique, chlorure de méthylène, tétrahydrofurane, éther diisopropylique et leurs mélanges.

10. Procédé selon la revendication 9, dans lequel ledit solvant est un mélange d'alcool méthylique et d'eau.

11. Procédé selon la revendication 1, dans lequel l'étape (b) de déshalogénation est effectuée à une température de 50 à 70°C.

12. Procédé selon la revendication 1, dans lequel l'étape (b) est effectuée avec un hydrure organostannique en présence d'un initiateur de radicaux.

13. Procédé selon la revendication 12, dans lequel ledit hydrure organostannique est l'hydrure de tributylétain et ledit initiateur de radicaux est l'α,α'-azoisobutyronitrile.
